# EUROPEAN PATENT APPLICATION

(11) **EP 3 591 035 A1**
(43) Date of publication of application: **08.01.2020**
(21) Application number: 19152226.7
(22) Date of filing: 17.01.2019
(51) Int. Cl.: C12N 5/00, C07K 14/47, G16B 25/10, G01N 33/574, G01N 33/68, G16B 5/20

(54) **FUNCTIONALIZED CULTURE SUBSTRATE TO MANIPULATE CELL FUNCTION AND DIFFERENTIATION**

(30) Priority: 06.07.2018 WO PCT/EP2018/068318
(71) Applicant: Koninklijke Philips N.V., 5656 AE Eindhoven (NL)
(72) Inventor: VAN DE STOLPE, Anja, 5656 AE Eindhoven (NL); BECKERS, Lucas Johannes Anna Maria, 5656 AE Eindhoven (NL)
(74) Representative: de Haan, Poul Erik

(57) **Abstract**

An *in vitro* method of producing a cell culture, tissue or organ comprises the steps of (a) determining first culture conditions that control activity of at least one first signaling pathway of cells to be cultivated, the activity of the at least one first signaling pathway being selected so to control cell behavior in a first predetermined manner; (b) cultivating the cells in a culture unit under the first culture conditions; (c) inferring activity of the at least one first signaling pathway in a cell sample obtained from the cell culture; and (d) comparing the inferred activity with the selected activity.

## Description

### FIELD OF THE INVENTION

The present invention generally relates to the field of biotechnology, bioinformatics and related arts. More particularly, the present invention relates to an in vitro method of producing a cell culture, tissue or organ. The present invention further relates to a non-transitory storage medium storing instruction that are executable by a digital processing device to perform the aforementioned method. The present invention further relates to a computer program comprising program code means for causing a digital processing device to perform the aforementioned method, when the computer program is run on a digital processing device. The present invention further relates to a system for performing the aforementioned method.

### BACKGROUND OF THE INVENTION

Cell function and differentiation during embryonic development, in adult life, and in disease, is controlled and steered by around 15 signal transduction pathways, like for example the Hedgehog, PI3K and estrogen receptor pathways. Pathway activity is regulated by cell-extrinsic factors among others, including environmental stimuli, such as small molecules, secreted proteins, temperature, and oxygen. These stimuli can originate from other cells within the organism, or they can come from the organism's environment. Within the organism, cells communicate with each other by sending and receiving secreted proteins, also known as growth factors, morphogens, cytokines, or signaling molecules. Receipt of these signaling molecules triggers intercellular signaling cascades that ultimately cause changes in transcription or expression of genes. This process is thought to regulate a vast number of cell behaviors.

In higher organisms, intercellular signaling pathways have the important task of coordinating and regulating cell division. The pathways ensure that cells divide synchronously and, if necessary, arrest cell division and enter a resting state. Cellular communication assumes great importance in the differentiation and development of an organism. The development of an organism is based on genetic programs that always utilize inter- and intracellular signaling pathways. Signal molecules produced by one cell influence and change the function and morphology of other cells in the organism. Intercellular signaling pathways are also critical for the processing of sensory information. External stimuli, such as optical and acoustic signals, stress, gradients of nutrients, and so on, are registered in sensory cells and are transmitted to other cells of the organism via intercellular signaling pathways. Naturally, a variety of timely and spatially controlled stimuli are provided to regulate cellular behavior.

For example, during embryonic development, stem cells differentiate into all kinds of body cells. The stem cells go through multiple defined differentiation steps to finally mature into the fully differentiated cell, like a skin cell, neuron, etc. During adult life stem cells and progenitor cells reside in organ niches under highly specific conditions and differentiate to replace organ cell types that are lost due to physiological processes (e.g. in the intestinal mucosa) or due to disease-associated organ damage. In benign and malignant tumors cells have lost differentiation features, allowing them to start dividing and metastasizing; this is again orchestrated by (spatiotemporal) activity of the signal transduction pathways. All this is orchestrated by highly controlled (spatiotemporal) activity of signal transduction pathways.

In other diseases, there is often abnormal activity of signal transduction pathways, which plays a causal role in the disease, in its progression and response to therapy.

Investigation of function of cells and tissues or of a specific disease to better understand (patho-)physiology and to develop drugs and therapies (e.g. regenerative medicine) requires *in vitro* cell and tissue culture model systems for healthy and/or diseased cell/tissue. The success of these and other *in vitro* applications that reside on cells to acquire a particular cellular behavior largely depends on the conditions in the immediate vicinity of the cells. Physical, chemical, and biological control of cell microenvironment are thus considered to be of crucial importance for the ability to direct and control cell behavior spatially and temporally in clinically associated application such as the production of 3-dimensional tissue engineering scaffolds.

A precise control of multiple stimuli in the cell culture microenvironment, which regulate intracellular signaling and ultimately cell phenotype, is particularly important in stem cell culture and differentiation (but similarly important to other cultures). Recapitulating the *in vivo* cells/tissue by *in vitro* culture is possible, on the premise that the required stimuli (stimuli) are provided in the correct spatiotemporal manner. The stimuli may consist of ligands, mostly proteins, that bind to and activate specific cell membrane receptors, to activate certain intracellular signal transduction pathways. Activation of such a pathway leads to a functional change in the cell. Moreover, the culture surface should mimic the natural in vivo cellular environment, which is a hurdle to optimally and most efficiently differentiating and maturing stem cells in the required directions.

For already differentiated cells it is generally more important to influence the function of the cells in a controlled manner, like cell division, migration, metabolism and the like. However, the same requirements as given for the differentiation of stem cells apply.

While it is difficult for conventional culture systems to provide such an accurate control *in vitro,* microfluidic devices are thought to provide a solution to this problem. For example, in a review of Zhang et al, published in Future Sci. OA, 2017, 3(2), FSO187, it is explained that microfluidic devices are ideally suited for stem cell culture and maintenance by providing the stimuli to create an *in vivo* like microenvironment. Advantageously, microfluidic devices offer the possibility to facilitate stimulation of cells by both biochemical stimuli and structural stimuli including well-defined surface features and patterned scaffolds, mechanical stress and electromagnetic forces, among others. Moreover, microfluidic devices can control multiple stimuli simultaneously over space and time with high precision and may thereby provide an ideal and well-defined platform for stem cells.

A disadvantage of known techniques, whether carried out in conventional culture systems or microfluidic devices, is that they rely primarily on control of culture conditions. The term control as used by various authors including Zhang et al, is to be understood to describe generally the possibility to tune culture conditions to provide a microenvironment suitable for the respective purpose. The selection of suitable culture conditions is based on existing knowledge and/or extensive experience by the experimenter. With these methods the results are heavily dependent on the level of existing knowledge and/or experience.

It would therefore be desirable to have methods that allow a control of culture conditions that is more robust, in particular less independent from existing knowledge and/or experience.

### SUMMARY OF THE INVENTION

In accordance with a first aspect of the present invention, the above problem is solved by a method of producing a cell culture, tissue or organ. The method comprises the steps of:
(a) determining first culture conditions that control activity of at least one first signaling pathway of cells to be cultivated, the activity of the at least one first signaling pathway being selected so to control cell behavior in a first predetermined manner;
(b) cultivating cells in a culture unit under the first culture conditions;
(c) inferring activity of the at least one first signaling pathway in a cell sample obtained from the cell culture; and
(d) comparing the inferred activity with the selected activity.

The inventors of the present invention found that despite of steadily increasing knowledge and experience with regard to stimuli required for a cell to acquire a particular cell behavior, it is difficult to consistently steer cells into the same direction. The level of knowledge and/or experience has a direct effect on the obtained result. If corrections to the culture conditions during cultivation are made, then these are based on deviations of actual culture condition from the culture conditions which according to existing knowledge and/or experience lead to the desired cell behavior. It is not taken into account that the existing knowledge and/or experience may be insufficient or incorrect and hence that the culture conditions to be assumed to be suitable are sub-optimal or even wrong.

With the present invention a robust method is provided that can be controlled so that cells may reproducibly adopt a particular cellular behavior that is less dependent from existing knowledge and/or experience as known methods. The method of the present invention allows to directly determine whether the cells indeed react to the culture conditions determined in step (a) in the expected manner. Therefore, in the first step (a) culture conditions are determined that are known or at least assumed to steer a particular cell (i.e. cell type) towards a desired cell behavior. In step (b), cells of the same cell type as in (a) are cultivated to provide a cell culture. Pathway activity is inferred in step (c). Comparing the inferred activity with the activity selected so to control cell behavior in a first predetermined manner (i.e. the selected activity) of the at least one first signaling pathway during cultivation allows to monitor cell response on the level of the cell culture, monitor progress of the cell culture to acquire a particular cellular behavior, confirm or decline that the cell culture has acquired the desired cell behavior or steer into the desired direction, and the like. More particularly, the present invention allows to draw conclusions with regard to cell behavior by preferably quantitatively inferring activity of signaling pathway(s) that mediate the respective cell behavior. For example, differentiation of stem cells to certain tissue may involve activation of one or more pathways. Inferring activity of said one or more pathways allows to safeguard that the respective signaling pathway(s) is/are indeed active. Information, in particular quantitative information, on the pathway activity can be advantageously used for the purposes of the present invention as a basis for minimizing deviations from the desired cell behavior so that cell behavior can be precisely controlled, and cells can be steered into the desired direction. The present invention thus provides a method which enables cell cultivation in a more controllable fashion than it is possible with existing methods.

According to a second aspect, the problem is solved by a non-transitory storage medium storing instruction that are executable by a digital processing device to perform the method of the invention.

According to a third aspect, the problem is solved a computer program comprising program code means for causing a digital processing device to perform the method of the invention, when the computer program is run on a digital processing device.

According to a fourth, aspect the problem is solved a system for performing the method of the invention, the system comprising
(a) a culture unit,
(b) a sensor unit for inferring an activity of at least one first signaling pathway;
(c) a controller that is configured to compare the inferred activity with a selected activity of the at least one first signaling pathway, the selected activity being selected so to control cell behavior in a first predetermined manner of the invention or a computer program of the invention.

Further advantages will be apparent to those of ordinary skill in the art upon reading and understanding the detailed description provided herein below. This application describes several preferred embodiments. Modifications and alterations may occur to others upon reading and understanding the preceding and following description. It is intended that the application is construed as including all such modifications and alterations insofar as they come within the scope of the appended claims or equivalents thereof.

Other variations to the disclosed embodiments can be understood and effected by those skilled in the art in practicing the claimed invention, from a study of the disclosure, and the appended claims.

It shall be understood that the method of claim 1, the non-transitory storage medium of claim 12, the computer program of claim 13 and the system of claim 14 have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the internet or other wired or wireless telecommunication systems.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWING

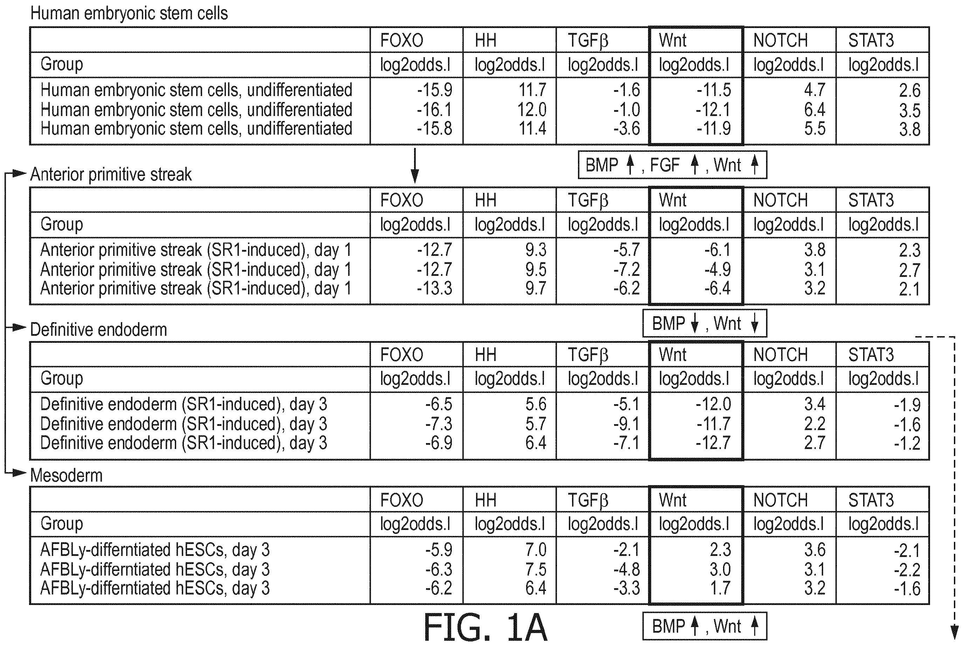

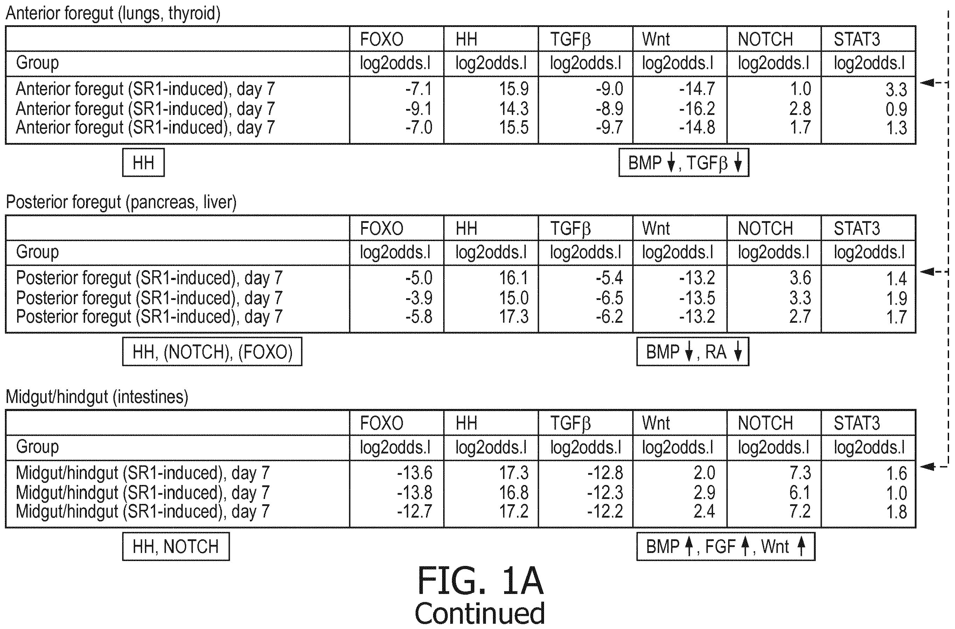

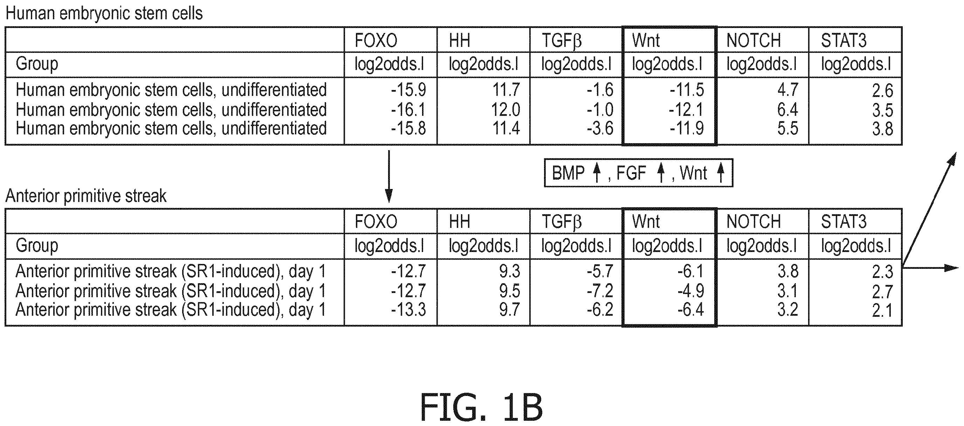

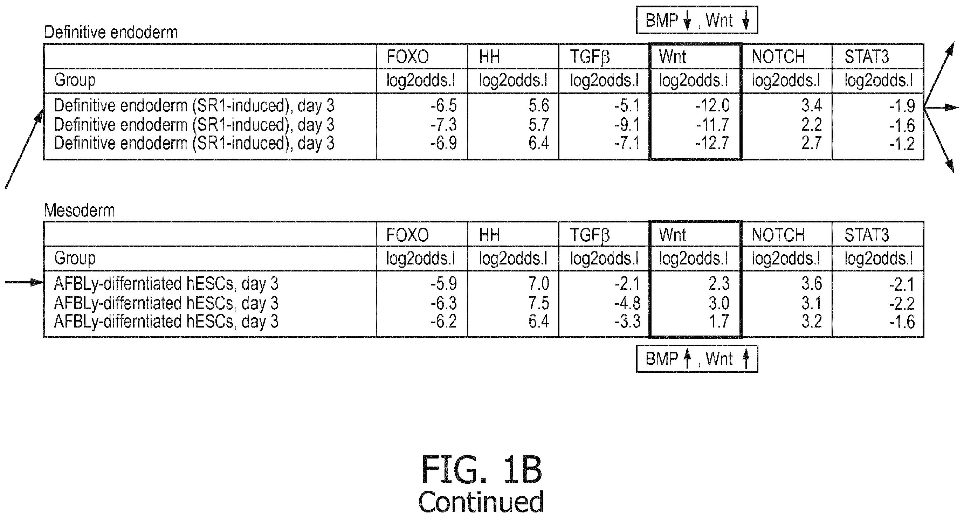

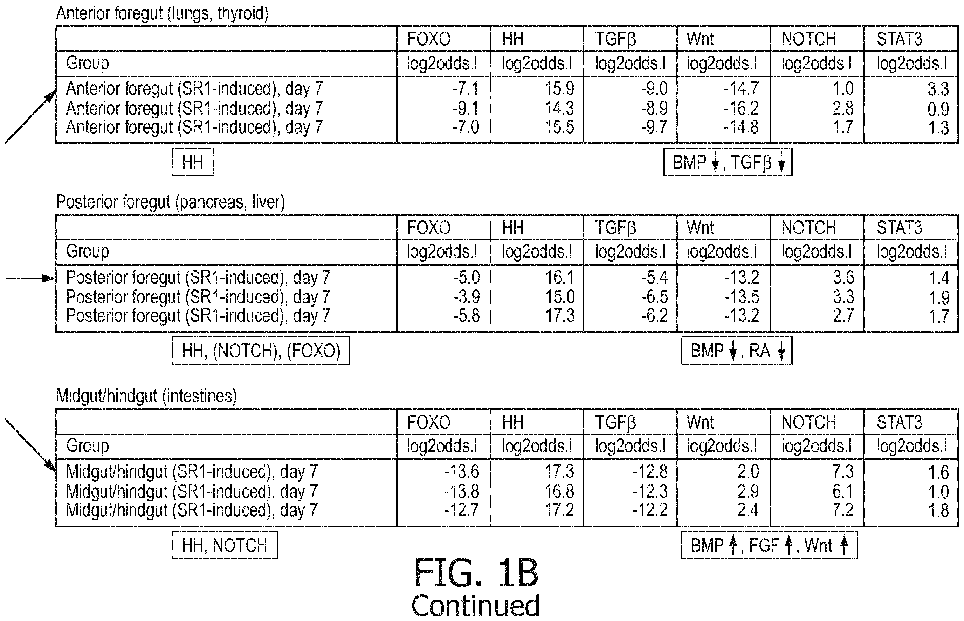

Fig. 1 exemplarily illustrates pathway activity during differentiation of human stem cells to endoderm (obtained by Philips pathway analysis on Affymetrix U133 Plus2.0 microarray data available in the publicly available GEO dataset GSE52658).
   A. Philips pathway activities measured per differentiation step are indicated next to the annotation (e.g. anterior primitive streak, definitive mesoderm, midgut/hindgut, etc.) per individual sample, as log2odds score of the respective pathway activity, respectively PI3K-FOXO, Hedgehog (HH), TGFbeta, Wnt, Notch, STAT3 pathways; names of the pathways are listed in the box on top of the activity scores.;
   B. Overview picture showing sequential differentiation steps during which specific pathways are activated, necessary to obtain the respective step in differentiation; signaling pathway activities are indicated as log2 odds values per individual sample for respectively PI3K-FOXO, Hedgehog, TGFbeta, Wnt, Notch, STAT3 pathways. Human embryonic stem cells (hESC) can be differentiated into various cell-types under the control of specific signals. Signaling pathway activity is a readout of such signals and measuring pathway activity can be a useful tool to assess the differentiation/maturation status of cultured cells. hESCs can be differentiated into various cell types (in this example in Figure 1B: anterior/posterior midgut and hindgut) by passing through various differentiation steps (in this example in Figure 1B: anterior primitive streak and definitive endoderm), as depicted by arrows in Figure 1B. For example, anterior foregut is formed from anterior primitive streak via definitive endoderm. Signaling pathway activity was measured using Philips signaling pathway analysis for each cell type, resulting in specific pathway signatures.

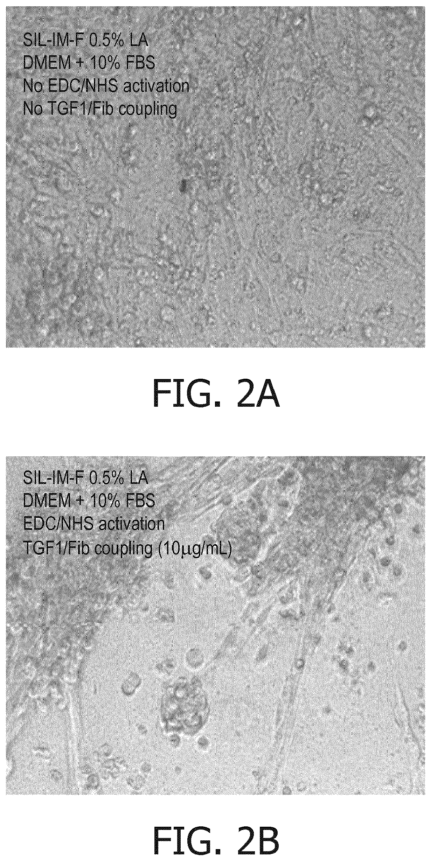

Fig. 2 exemplarily shows the results of a differentiation experiment carried out on non-functionalized surface (A.) and functionalized surface coated with TGFb1 (B.), on which WPMY-1 cells were seeded. Cells bind in a specific manner to the TGFbeta-coated surface, which binding is assumed to be dependent on the cells' expression of TGFbeta receptors. On the non-coated surface cells bind in a specific manner, independent of expression of specific receptors, like the TGFbeta receptor. Fewer cells bind to the TGFbeta-coated surface, and cell morphology changes, probably caused by the TGFbeta induced activation of the TGFbeta signal transduction pathway (see experiment using TGFbeta as an example described further below).

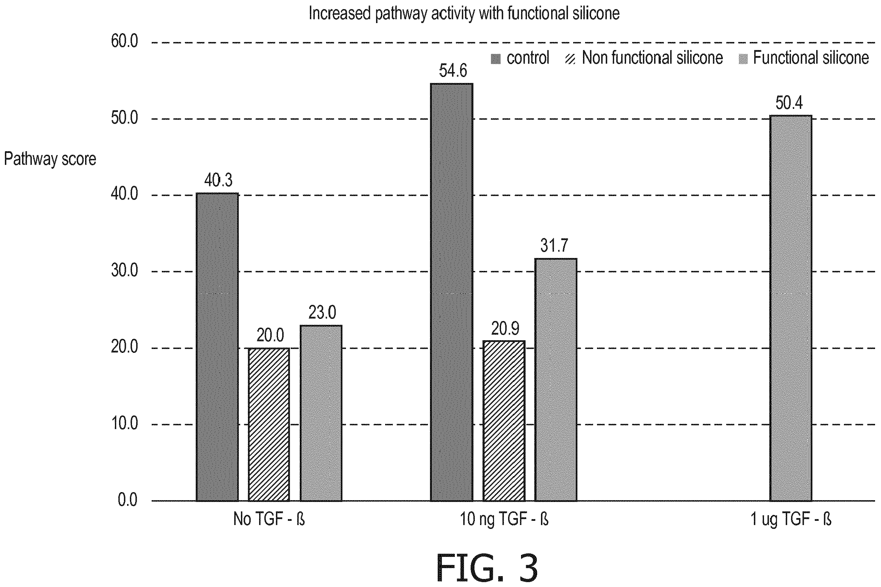

Fig. 3 exemplarily shows an independent cell culture experiment in which the concentration of TGFb1 has been varied on 3 different surfaces. A tissue culture-treated plastic surface, a non-functionalized and a functionalized surface were incubated with different concentrations of TGFb1, 0 ng/ml, 10 ng/ml and 1000 ng/ml (indicated in Fig. 3 as respectively NO, 10 ng and 1 microgram; on the y-axis is shown the TGFbeta pathway activity score).

### DETAILED DESCRIPTION OF EMBODIMENTS

The following embodiments merely illustrate particularly preferred methods and selected aspects in connection therewith. The teaching provided herein may be used for constructing several test methods, test systems and/or test kits, e.g., to produce a cell culture, tissue and/or an organ. The following embodiments are not to be construed as limiting the scope of the present invention.

The extracellular microenvironment plays a significant role in influencing cellular behavior including maintenance of potency, cellular attachment, proliferation, metabolization, lineage-specific differentiation and remodeling. By manipulating the culture conditions in which cells including stem cells are cultivated, it is possible to influence cell behavior, restrict differentiation pathways and thereby produce cell cultures enriched in lineage-specific precursors, particular tissues or organs in vitro among others. Based on this understanding, the method of the first aspect of the invention comprises the step (a) of determining first culture conditions that control activity of at least one first signaling pathway of cells to be cultivated. The activity of the at least one first signaling pathway is thereby selected so to control cell behavior of the cells to be cultivated in a first predetermined manner. The determination in step (a) does not imply an active experimental step but may simply mean that information is gathered, e.g. based on in silico predictions, existing knowledge (for example published literature and/or data banks) and/or experience, which signaling pathways are active or inactive in the cells (cell type) of interest in order such cells acquire a particular cellular behavior. For example, it is known that differentiation of certain stem cells towards specific cell types or tissue involves a cascade of cell signaling (cf., e.g. Tsai et al, BMC Cell Biol, 2010, 11:76). Based on this knowledge, it can be determined that these signaling pathways should be active or inactive in a sequential manner to arrive at the desired progeny.

The term "cell behavior" or "cellular behavior" may denote a functional or morphological response of a cell or cell culture to the culture conditions applied. Typical functional responses that may be induced include, for example, cell division, cell growth, differentiation, development, maintenance of pluripotency, etc. Further cell behaviors that can be controlled by the present method are described herein.

The terms "pathway", "signal transduction pathway" and "signaling pathway" are used interchangeably herein. The at least one signaling pathway may mediate cell behavior of the cells to be cultivated.

The term "inferring activity" in the context of pathway activity herein implies a step in which a dimension corresponding to the pathway activity is inferred. The dimension may preferably describe the pathway activity in a semi-quantitative manner, wherein the activity may be classified according to, for example, no activity, low activity, high activity, and the like, or in a quantitative manner, wherein the activity is expressed in the form of discrete or continuous values, the values corresponding to levels of activity. In this way, a semi-quantitative or quantitative comparison in step (d) is possible. The comparing in step (d) allows to determine whether the inferred activity corresponds to the selected activity so as to control cell behavior in the predetermined manner. It can therefore be determined whether the cultivated cells behave in the desired manner, e.g. differentiate to the desired progeny, maintain pluripotency or the like, and if so the culture conditions may be maintained or if not they may be changed so as to correct any observed deviation from the desired behavior reflected by the pathway activity determined in step (a).

According to a preferred embodiment, the method further comprises step (e) of determining second culture conditions based on the comparison of the inferred activity with the selected activity. Preferably, the second culture condition is determined so as to (i) reduce deviation of the inferred activity from the selected activity. This means, in case the inferred activity of the at least one signaling pathway does not correspond to the expected activity, i.e. the activity determined in step (a), the second culture conditions determined in step (e) may reflect conditions that are expected to correct any observed deviation from the desired behavior reflected by the pathway activity determined in step (a).

Alternatively, the second culture conditions may be determined so as to (ii) control activity of at least a second signaling pathway, the activity of the at least one second signaling pathway being selected so to control cell behavior in a second predetermined manner. For example, the inferred activity corresponds to the expected activity and the second culture conditions determined in step (e) may be substantial identical to the first culture conditions determined in step (a). This is useful in case the cell culture is intended to continue to divide and/or maintain pluripotency. According to another example, the inferred activity corresponds to the expected activity and the second culture conditions are determined in step (e) to control activity of at least one second signaling pathway of the cells, the activity of the at least one second signaling pathway being selected so to control cell behavior in a second predetermined manner. This may be particularly useful, when differentiation of the cells to a particular progeny involves a cascade of signaling pathways that needs to be activated or deactivated in a sequential manner.

According to a further preferred embodiment, the method further comprises the following steps (f) cultivating the cells in the culture unit under the second culture conditions; and (g) repeating steps (c) and (d) as described herein. Preferably, the steps (c) and (d) are repeated in intervals. The intervals may be set depending on the cells, cultivation conditions and/or desired cell behavior and may range from a few hours to several weeks. Suitable intervals for example range from 6 hours to 3 weeks, 6 hours to 2 weeks, 6 hours to 10 days, 6 hours to 7 days, preferably 8 hours to 5 days, more preferably 8 hours to 3 days, in particular 8 hours to 2 days such as once daily.

Optionally, the method further comprises step (h) determining third culture conditions based on the comparison of the activity inferred in step (g) with the selected activity. In this manner, the method enables feedback control of the cell behavior based on pathway activity. The optional step (h) may for example reflect conditions to influence activity of at least one third signaling pathway, for example a third signaling pathway involved in a cascade of multiple signaling pathways required for differentiation of the cells to a particular progeny.

With the method of the present invention cell behavior that is mediated by one or more signaling pathways can be controlled. The cell behavior that can be influenced is not particularly limited and includes differentiation, maintenance of pluripotency and cellular function, such as cell division, migration and metabolism, chemotaxis, adhesion, apoptosis, generating oxidative stress, DNA repair, production of secretory proteins, production of extracellular matrix proteins and the like.

### Stimuli

According to a preferred embodiment, the first, second and/or third, etc., culture conditions are defined by controlled exposure of the cells to one or more stimuli, wherein the stimuli control activity of the at least one first and/or second signaling pathway. In other terms, the cell culture is exposed to well-defined stimuli that may activate or deactivate the respective signaling pathway(s). Suitable stimuli include both naturally occurring substances but also non-natural substances such as artificial proteins or peptides, drugs that activate or modify pathway activity by binding to a surface receptor, such as antibodies like trastuzumab and antigen peptides that bind to T cell or B cell receptors and initiate cell division through the PI3K pathway. Accordingly, it is preferred that the one or more stimuli are selected from the group of cell receptor ligands, extracellular matrix molecules, artificial receptor agonists or antagonists, or specific antigens for T or B cell receptors.

According to a preferred embodiment, the one or more stimuli are coupled to a surface of the culture unit. Coupling the one or more stimuli to a surface in the correct three-dimensional conformation in the immediate microenvironment of the cells more closely reflect *in vivo* situation, where ligands are often expressed on the outside of cell membrane of an adjacent cell or bound to the extracellular matrix surrounding the cells, than by providing them in solution flowing along the cells attached to the surface. This embodiment may render many ligands very effective.

The one or more stimuli are preferably provided in a definite spatial and/or temporal manner. To provide the one or more stimuli in a particular spatial distribution the surface may be patterned with different types of stimuli. For example, two different cell types with different stimuli requirements may be aligned. In addition, the concentration of stimuli may be varied and/or adapted to the particular need. For example, a low concentration of a particular stimuli may induce cell differentiation, whereas a high concentration of the same stimuli induces cell division. The TGF-β pathway as a typical example dictates cell fate in a concentration-dependent manner. In addition or alternatively to variations of the concentration, concentration gradients may be envisaged. Mixtures of stimuli may be provided when different receptors need to be activated to obtain the desired effect in the cells. An example would be use of a growth factor ligand in combination with TGFbeta.

### Surface properties and chemistry

Most of large-sized tissues and organs with distinct three-dimensional form will require support for their formation from cells. For example, stem cells reside in a specialized microenvironment called stem cell niche which provides the stem cells with extracellular stimuli to allow their survival and identity. This niche is a key regulator to the stem cell behavior because it ensures a quiescent and low metabolic environment to prevent exhaustion. It is believed that microenvironmental properties of the niche provide a good balance between the ability of SCs to renew themselves and the ability to differentiate into mature cells so that continuous tissue regeneration occurs. A major part of the cell niche is the ECM (extracellular matrix) which possesses the specific mechanical, biochemical, and biophysical properties for tissues and controls the overall cell behavior. The composition of the ECM provides full support to the niche through its physical and structural properties. The major function of the artificial support is similar to that of the natural ECM that assists in proliferation, differentiation, and biosynthesis of cells. For this reason, it is desired that the surface of the culture unit not only comprises biochemical stimuli but also has definite physical properties such as definite mechanical properties including surface stiffness/flexibility.

According to a preferred embodiment, the surface comprises or consists of a polymer-based material, preferably based on polydimethylsiloxane (PDMS) or Lucas rubber (polybutadiene), and wherein the one or more stimuli are coupled to the surface via carboxyl groups. Advantageously, coupling the one or more stimuli to carboxyl groups sticking out of surface material such as PDMS or Lucas rubber mimics in vivo situation for example for cell receptor ligands of members of the TGFbeta family, ligands of the Hedgehog and Notch pathways, and ligands of integrin-induced cell signaling. As a further advantage, variations in substrate stiffness can be easily obtained with both rubber and PDMS.

According to a preferred embodiment, the surface comprises or is composed of a material for binding said one or more stimuli such as cell receptor ligand, wherein the material corresponds to a material for binding to a cell culturing protein as described in international patent application PCT/EP2018/068318, titled "Cell culturing materials", having a filing date of 6 July 2018 and claiming priority of EP application number 17181708.5 filed on 18 July 2017, which disclosure is herein incorporated by reference in its entirety. The therein disclosed material is capable of binding the herein described one or more stimuli in place of the cell culturing protein described in the afore-said international patent application.

For the purposes of the present invention, the material in particular contains a bulk-modified elastomer comprising a plurality of fatty acid moieties covalently bound to the elastomer bulk, wherein the carboxylic acid groups of said moieties are available to provide binding to the one or more stimuli. Such a material can advantageously be manufactured in a few steps. In some embodiments, a single step suffices, for example when the manufacturing is carried out by combining the elastomer and the fatty acid in a coating process (e.g., spin coating, dip coating, spray coating, dispensing) or an injection molding process in which the cross-linking of the elastomer with the fatty acid carbon-carbon double bond can be achieved without significant epoxidation of the fatty acid carbon-carbon double bond due to the limited exposure to ambient oxygen in the coating or injection molding process. Thereby, a material is provided in which the elastomer is bulk modified with fatty acid moieties in which the carboxylic acid groups of the fatty acid are available to bind to a biocompatible material such as the one or more stimuli described herein.

Moreover, by controlling curing and/or properties of the mold in which the material is formed, such carboxylic acid groups can predominantly present on an outer surface of the material, thereby providing a substantially homogeneous distribution of carboxylic acid groups on such outer surface, which makes the material particularly suitable for use as a membrane material for a fluidic device, as each cross-section of the material will exhibit the same surface properties, in contrast to membrane materials onto which an anchor for the biocompatible material needs to be grafted or otherwise formed as with known techniques. In addition, because typically only a fraction of the elastomer carbon-carbon double bonds is consumed in such a cross-linking reaction, the material retains the elastomeric properties of the elastomer, which adds to the suitability of the material for use in fluidic devices and facilitates the slicing or otherwise cutting of the material for investigative or other purposes.

Preferably, each of the fatty acid moieties is covalently bound to the elastomer bulk through a cross-linking reaction between a vinyl functional group or a hydride functional group of the elastomer and an unsaturated carbon-carbon bond of an unsaturated fatty acid to ensure that the number of carboxylic acid groups available for binding to the biocompatible material can be optimized.

The elastomer may be any suitable elastomer. For example, the elastomer may be a homopolymer, a copolymer, a block copolymer, a terpolymer, a block terpolymer and so on. A particularly suitable elastomer may be selected from polyenes such as polybutadiene or silicones (polysiloxanes). Such silicones may comprise a PDMS backbone including vinyl moieties to facilitate cross-linking through Pt-catalyzed addition reactions, e.g. with (poly methyl) hydrogen siloxanes. Alternatively, a silicone backbone such as a (poly methyl) hydrogen siloxane backbone may be crosslinked with rubber-like polymers such as polybutadiene and polyisoprene, with the unsaturated fatty acid being incorporated in such a crosslinked product.

Regarding to unsaturated fatty acid, any suitable unsaturated fatty acid may be used for the purpose of cross-linking it with the elastomer. For example, the unsaturated fatty acid may be selected from myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid and docosahexaenoic acid. Linoleic acid is specifically mentioned.

The elastomer and the unsaturated fatty acid may be mixed in any suitable ratio in a composition to form the bulk-modified elastomer. Preferably, unsaturated fatty acid is present in such a composition in a range of 0.05-35% by weight of the elastomer such that upon bulk modification of the elastomer the bulk-modified elastomer retains its flexibility to the remaining presence of carbon-carbon double bonds in the elastomer bulk.

The material comprising (i.e. binding) the one or more stimuli may be denoted as cell culturing scaffold in correspondence to the terminology used in the aforementioned international patent application. Such a cell culturing scaffold can be manufactured in a straightforward manner by a simple binding reaction of the one or more stimuli to the carboxylic acid groups of the material, thereby leading to a cell culturing scaffold having a substantially homogeneous distribution of the one or more stimuli across a surface of the material.

If a patterning of stimuli as described above is desired, a fluidic device with more than one membrane may be used, wherein each membrane can be advantageously manufactured as described above. The membranes may be stacked so that cell can be cultivated in front of each other. In this way, for example liver tissue and pancreas tissue can be grown in front of each other. The consumption of glucose in the nutrition for both organs and the production of insulin by the pancreas could be studied in a co-working organ on chip system.

### Microfluidic devices

Control of cellular behavior such as stem cell culture and differentiation require precise control of multiple stimuli in the cell culture microenvironment, which regulate intracellular signaling and ultimately cell phenotype. Microfluidic devices, which can control multiple stimuli simultaneously over space and time with high precision, provide an ideal and well-defined platform for cultivating cells such as stem cells in a controlled manner.

According to a preferred embodiment, the cell culturing scaffold is part of a fluidic device module including at least one flow channel extending over a membrane, the membrane comprising said cell culturing scaffold. That is, the culture unit is a microfluidic device. Such a fluidic device module may comprise a first major surface comprising a first recessed structure defining a first flow channel. Depending on the application, a second major surface opposing the first major surface and comprising a second recessed structure defining a second flow channel may be foreseen with the membrane separating the first flow channel from the second flow channel. This is particular useful in case the cell culture shall be later used in the same device to monitor the cells' response to a drug as described in the aforementioned international patent application. In a preferred embodiment, the fluidic device module is a monolithic fluidic device module, which has the advantage that the device module can be manufactured in a small number of processing steps, e.g. a single processing step, by injection molding.

The membrane may comprise a plurality of holes or grooves extending through the membrane. The holes or grooves have preferably cell-size dimensions in order to retain the cells to be cultured.

In an embodiment, the first flow channel and the second flow channel are accessible through respective septa, such that the separate flow channels may be individually accessed without cross-contamination risk.

The fluidic device module may further comprise a pair of cover plates (e.g. transparent glass sheets), wherein one cover sheet comprises preferably two inlets and two outlets, to fluidly seal the fluidic device module. The cover plates may be arranged such that one of said cover plates covers the first major surface, thereby sealing the first fluidic channel and the other of said cover plates covers the second major surface, thereby sealing the second fluidic channel. Such a fluidic device can be manufactured in a straightforward manner, as the fluidic device module may be formed in a small number of processing steps as previously explained and is robust against leakage due to the flexible nature of the fluidic device module. If the membrane is made of an elastomer material, in particular a soft flexible elastomer material, the cover plates advantageously forms together with the membrane clamped between the cover plates a leakage tight fluidic device module.

In a preferred embodiment, the membrane is capable of adhering to the top and/or bottom cover plates. Consequently, after clamping the membrane and the cover plates together, said parts stay together due to the membrane's sticky properties. Thereby, it is possible to handle the fluidic device module under/on the microscope such as a confocal microscope after having the fluidic device module removed from a fluidic system like Micronit or Fluigent without risk that the cover plates, being in particular made of a transparent material to facilitate microscopy, detach from the membrane.

It is also envisaged that the membrane allows the top cover plate to be easily removed, whereas it adheres to the bottom cover plate so that the cell culture, organ, tissue or biopsy on the membrane can be bended towards the bottom cover plate. Thereby, the bottom cover plate remains in touch with lower side of the membrane and allows the membrane to be positioned precisely in focus of a confocal microscope.

In order to change culture conditions from the first culture conditions to the second culture conditions, etc. at different times, the cells grown on a first surface and/or membrane may be detached and replaced by a second surface and/or membrane comprising one or more second stimuli. Advantageously, a plurality of different stimuli-precoated surfaces and/or membranes may be prepared and provided in a kit format for this purpose.

For a detailed description of suitable manufacturing methods, further suitable unsaturated fatty acids and elastomers, particulars with regard to structural modifications of the membrane and/or configurations of the fluidic device module, the reader is referred to the above-cited international patent application.

### Cell types

The cell is not particularly limited and can be any cell or cell line that is intended to acquire a particular cellular behavior. According to a specific embodiment, the cells are selected from the group consisting of mouse stem cells, human stem cells, pluripotent stem cells, mesenchymal stem cells, embryonic stem cells, adult stem cells and tumor cells.

Stem cells have enormous potential in numerous clinically related applications including tissue engineering, organ regeneration, cell-based therapies, disease models, drug response testing, drug screening and development and a variety of healthcare applications. The primary step in most applications involving stem cells is to direct the differentiation of the cells to the desired progeny. Naturally, there are two types of stem cells, the first being embryonic stem cells (ESCs) and the second being adult stem cells. ESCs are isolated from the inner cell mass of blastocysts and have the pluripotency to differentiate into virtually all cell lineages. Adult stem cells can be found in various tissues and can differentiate to a limited number of cell types. In addition, there are reprogrammed human-induced pluripotent stem cells (hiPSCs), which can be directly obtained from adult cells by introduction of specific genes. Because they can propagate indefinitely, as well as give rise to every other cell type in the body, they represent a single source of cells that could be used to replace those lost due to damage or disease. In certain embodiments, the cells employed in the present method do not require destruction of an embryo. In such certain embodiments, embryonic stem cells are excluded from the scope of the invention.

Tumor cells may be used for producing a cancer model system, for the purposes of diagnosing or subtyping a cancer and/or testing response of the cancer cells to drugs.

### Pathway analysis

According to a further preferred embodiment, the at least one signaling pathway is selected from the group of:
nuclear receptors, like ER, AR, progesterone receptor;
growth factor pathways, like PI3K-FOXO, JAK-STAT3, MAPK-AP1 pathways;
immune pathways like STAT1/2 type I interferon (STAT1/2-1), STAT1/2 type II interferon (STAT1/2-2) pathways;
developmental pathways, like Hedgehog, Notch, TGFbeta, and Wnt signaling pathways; and
the inflammatory pathway NFkB.

According to a further preferred embodiment, the activity of the at least one first and/or second, etc., signaling pathway in the cell sample is inferable by a method comprising:
receiving expression levels of three or more target genes of the at least one signaling pathway in at least one cell contained in the cell sample,
determining an activity level of a transcription factor (TF) element in the at least one cell, the TF element controlling transcription of the three or more target genes, the determining being based on evaluating a calibrated mathematical pathway model relating expression levels of the three or more target genes to the activity level of the at least one signaling pathway, and
inferring the activity of the at least one signaling pathway in the at least one cell based on the determined activity level of the TF element, wherein the calibrated mathematical pathway model is preferably a centroid, linear or Bayesian network model, based on conditional probabilities.

The term "activity of the at least one signaling pathway" may refer to the activity of a signaling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or respective values, levels, scores, dimensions or the like related to or indicative for such activity (e.g. speed). Accordingly, for the purposes of the present invention, the term "activity", as used herein, is also meant to refer to said activity level. Both the activity and the activity level can be determined by pathway analysis as described herein. As described above, the activity may be identical to the activity level. Such level can be directly used as an input for step (d).

The pathway activity is determinable, e.g., in a sample isolated from the cell culture, by pathway analysis as described herein.

Pathway analysis enables quantitative measurement of signal transduction pathway activity in cells present in tissue/cell samples, based on inferring activity of a signal transduction pathway from measurements of mRNA levels of the well-validated direct target genes of the transcription factor associated with the respective signaling pathway (see for example W Verhaegh, et al., Selection of personalized patient therapy through the use of knowledge-based computational models that identify tumor-driving signal transduction pathways. Cancer research 2014;74(11):2936-45; W Verhaegh, A van de Stolpe. Knowledge-based computational models. Oncotarget 2014;5(14):5196).

According to a preferred embodiment of the first, second, third and fourth aspect of the present invention and the various embodiments thereof, the determining of the activity of one or more pathways, the combination of multiple pathway activities and applications thereof is performed as described for example in the following documents, each of which is hereby incorporated in its entirety for the purposes of determining activity of the respective signaling pathway: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES") and in the patent applications EP16200697.7 (filed on Nov 25, 2016; titled "Method to distinguish tumor suppressive FOXO activity from oxidative stress"), EP17194288.1 (filed on Oct 2, 2017; titled "Assessment of Notch cellular signaling pathway activity using mathematical modelling of target gene expression"), EP17194291.5 (filed on Oct 2, 2017; titled "Assessment of JAK-STAT1/2 cellular signaling pathway activity using mathematical modelling of target gene expression"), EP17194293.1 (filed on Oct 2, 2017; titled "Assessment of JAK-STAT3 cellular signaling pathway activity using mathematical modelling of target gene expression") and EP 17209053.2 (filed on Dec 20, 2017, titled "Assessment of MAPK-AP1 cellular signaling pathway activity using mathematical modelling of target gene expression"). The models have been biologically validated for ER, AR, PI3K-FOXO, HH, Notch, TGF-β, Wnt, NFkB, JAK-STAT1/2, JAK-STAT3 and MAPK-AP1 pathways on several cell types. It is noted that the mathematical models employed in the patent applications that are not yet published as well as the calibration and use of these models in these applications generally correspond to the models, calibration and use disclosed in the already published patent applications.

To facilitate rapid identification of references, the above-mentioned references have been assigned to each signaling pathway of interest here and exemplarily corresponding target genes suitable for determination of the signaling pathway's activity have been indicated. In this respect, particular reference is also made to the sequence listings for the target genes provided with the above-mentioned references.
**Wnt**: KIAA1199, AXIN2, RNF43, TBX3, TDGF1, SOX9, ASCL2, IL8, SP5, ZNRF3, KLF6, CCND1, DEFA6 and FZD7 (WO 2013/011479**,** WO 2014/102668**,** WO 2014/174003); ADRA2C, ASCL2, AXIN2, BMP7, CCNDl, CD44, COL18A1, DEFA6, DKKl, EPHB2, EPHB3, FAT1, FZD7, GLUL, HNF1A, CXCL8 (previously known as IL8), CEMIP (previously known as KIAAl 199), KLF6, LECT2, LEF1, LGR5, MYC, NKD1, OAT, PPARG, REGIB, RNF43, SLC1A2, SOX9, SP5, TBX3, TCF7L2, TDGF1, and ZNRF3 (WO 2016/062892**,** WO 2016/062893);
**HH**: GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1 (WO 2013/011479**,** WO2014/102668**,** WO2014/174003); GLI1, PTCH1, PTCH2, HHIP, SPP1, TSC22D1, CCND2, HI 9, IGFBP6, TOM1, JUP, FOXA2, MYCN, NKX2-2, NKX2-8, RAB34, MIF, GLI3, FST, BCL2, CTSL1, TCEA2, MYLK, FYN, PITRM1, CFLAR, IL1R2, S100A7, S100A9, CCNDl, JAG2, FOXM1, FOXF1, and FOXL1 (WO 2016/062892**,** WO 2016/062893);
**AR**: KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR and EAF2 (WO 2013/011479**,** WO 2014/102668); KLK2, PMEPA1, TMPRSS2, NKX3 1, ABCC4, KLK3, FKBP5, ELL2, UGT2B15, DHCR24, PPAP2A, NDRG1, LRIG1, CREB3L4, LCP1, GUCY1A3, AR, and EAF2 (WO 2014/174003);
**ER**: CDH26, SGK3, PGR, GREBl, CA12, XBP1, CELSR2, WISP2, DSCAM, ERBB2, CTSD, TFF1 and NRIPl (WO 2013/011479**,** WO 2014/102668); GREB1, PGR, XBP1, CA12, SOD1, CTSD, IGFBP4, TFF1, SGK3, NRIP1, CELSR2, WISP2, and AP1B1 (WO 2014/174003); AP1B1, ATP5J, COL18A1, COX7A2L, CTSD, DSCAM, EBAG9, ESRl, HSPBl, KRT19, NDUFV3, NRIPI, PGR, PISD, PRDM15, PTMA, RARA, SODl, TFFl, TRIM25, XBPl, GREBl, IGFBP4, MYC, SGK3, WISP2, ERBB2, CA12, CDH26, and CELSR2 (WO 2016/062892**,** WO 2016/062893);
**PI3K-FOXO**: AGRP, BCL2L11, BCL6, BNIP3, BTG1, CAT, CAV1, CCND1, CCND2, CCNG2, CDK 1A, CDK 1B, ESRl, FASLG, FBX032, GADD45A, INSR, MXIl, NOS3, PCKl, POMC, PPARGCIA, PRDX3, RBL2, SOD2 and TNFSF10 (WO 2015/101635); ATP8A1, BCL2L11, BNIP3, BTGl, ClOorflO, CAT, CBLB, CCNDl, CCND2, CDKNIB, DDB1, DYRK2, ERBB3, EREG, ESRl, EXT1, FASLG, FGFR2, GADD45A, IGF1R, IGFBP1, IGFBP3, INSR, LGMN, MXI1, PPM1D, SEMA3C, SEPP1, SESN1, SLC5A3, SMAD4, SOD2, TLE4, and TNFSF10 (WO 2016/062892**,** WO 2016/062893); SOD2, BNIP3, MXI1, PCK1, PPARGC1A and CAT (EP16200697.7, supra);
**TGF-β**: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1 and VEGFA (WO2016/062891**,** WO2016/062893);
**NFkB:** BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1 and VCAM1 (WO 2017/029215);
**Notch**: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9 and TNC (EP17194288.1, supra);
**JAK-STAT1/2**: BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1 and USP18 (EP17194291.5, supra);
**JAK-STAT3**: AKT1, BCL2, BCL2L1, BIRC5, CCND1, CD274, CDKNIA, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST 1, VIM and ZEB1 (EP17194293.1, supra);
**MAPK-AP1**: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53 and VIM (EP17209053.2, supra).

Common to the pathway analysis methods for determining the activities of the different signaling pathways as disclosed herein is a concept, which is preferably applied herein for the purposes of the present invention, wherein the activity of a signaling pathway in a cell present in a sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the three or more target genes to the activity level of the signaling pathway, and optionally inferring the activity of the signaling pathway in the cell based on the determined activity level of the signaling pathway associated TF element in the sample.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signaling pathway proteins, with one or more co-factors, thereby controlling transcription of target genes. Preferably, the term refers to either a protein or protein complex transcriptional factor triggered by the cleavage of one of the respective signaling pathway proteins resulting in an intracellular domain.

As indicated above, the term "activity level" of a TF element, as used herein, denotes the level of activity of the TF element regarding transcription of its target genes.

The term "target gene", as used herein, means a gene whose transcription is directly or indirectly controlled by a respective transcription factor element. The "target gene" may be a "direct target gene" and/or an "indirect target gene" (as described herein).

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a centroid or a linear model, or a Bayesian network model based on conditional probabilities.

In particular, the determination of the expression level and optionally the inferring of the activity of a signaling pathway in the cell sample may be performed, for example, by *inter alia* (i) evaluating a portion of a calibrated probabilistic pathway model, preferably a Bayesian network, representing the cellular signaling pathways for a set of inputs including the expression levels of the three or more target genes of the cellular signaling pathway measured in the cell sample, (ii) estimating an activity level in the cell sample of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the cell sample, and optionally (iii) inferring the activity of the cellular signaling pathway based on the estimated activity level of the signaling pathway associated TF element in the cell sample. This is described in detail in the published international patent application WO 2013/011479 A2 ("Assessment of cellular signaling pathway activity using probabilistic modeling of target gene expression"), the contents of which are herewith incorporated in their entirety.

In an exemplary alternative, the determination of the expression level and optionally the inferring of the activity of a cellular signaling pathway may be performed by inter alia (i) determining an activity level of a signaling pathway associated transcription factor (TF) element in the cell sample, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the cells of the cell culture based on the determined activity level of the signaling pathway associated TF element in the cell sample. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945.

In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

### Storage medium, computer program and system

In accordance with a second aspect of the present invention, a non-transitory storage medium stores instruction that are executable by a digital processing device to perform the method according to the first aspect of the invention, and the various embodiments thereof. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with a third aspect of the present invention, a computer program comprises program code means for causing a digital processing device to perform the method according to the first aspect of the invention, and the various embodiments thereof, when the computer program is run on the digital processing device. The digital processing device may be a handheld device (e.g., a personal data assistant or smartphone), a notebook computer, a desktop computer, a tablet computer or device, a remote network server, and so forth.

In accordance with a fourth aspect of the present invention, a system for performing the method of the invention comprises
(a) a culture unit,
(b) a sensor unit for inferring an activity of at least one first signaling pathway;
(c) a controller that is configured to compare the inferred activity with a predetermined activity of the at least one first signaling pathway, the predetermined activity being selected so to control cell behavior in a first predetermined manner of the invention or a computer program of the invention.

According to a preferred embodiment, the controller is further configured to determine culture conditions based on the comparison of the inferred activity with the selected activity, the culture conditions being optionally determined so as to (ii) reduce deviation of the inferred activity from the selected activity or (ii) control activity of at least one second signaling pathway, the activity of the at least one second signaling pathway.

The culture unit is preferably constituted as described herein in the context of the first aspect of the invention. The sensor unit is adapted to infer the activity of the at least one first signaling pathway. To this end, the sensor unit may be equipped with one or more components or means for measuring the expression levels of the target genes such as a DNA array chip, an oligonucleotide array chip, a protein array chip, an antibody, a plurality of probes, for example, labeled probes, a set of RNA reverse-transcriptase sequencing components, and/or RNA or DNA, including cDNA or amplification primers. Preferably, the sensor unit is adapted to perform one of qPCR, multiple qPCR, multiplexed qPCR, ddPCR, RNAseq, RNA expression array and mass spectrometry.

In an embodiment, the sensor unit uses a set of labeled probes directed to a portion of an mRNA or cDNA sequence of the target genes as described herein. In an embodiment, the sensor unit uses a set of primers and probes directed to a portion of an mRNA or cDNA sequence of the target genes. In an embodiment, the labeled probes are contained in a standardized 96-well plate. In an embodiment, the sensor unit further uses primers or probes directed to a set of reference genes. Such reference genes can be, for example, constitutively expressed genes useful in normalizing or standardizing expression levels of the target gene expression levels described herein.

The sensor unit may further include a processor to compute results corresponding to the inferred activity of the at least one first signaling pathway.

### Uses

The various aspects of the present invention are particularly useful for:
determining, predicting or controlling that a cell culture acquires a particular cellular behavior;
determining, predicting or controlling that a cell culture differentiated to or in the direction of the desired progeny;
determining, predicting or controlling that a cell culture acquires a particular function;
determining, predicting or controlling cell culture response to particular stimuli;
producing a cell culture, tissue or organ in a controlled and robust manner;
predicting, monitoring or determining response to therapy using the cell culture, tissue or organ produced by the method of the present invention;
predicting, monitoring or determining effectiveness of therapy using the cell culture, tissue or organ produced by the method of the present invention;
producing tumor model systems;
diagnosing or subtyping a disease, in particular tumor disease;
producing cells suited for regenerative medicine purposes;
producing series of cells that are very well defined in a quantitative manner with respect to their cellular state, e.g. differentiation state, to enable reproducibility subsequent experimental use, e.g. drug development or regenerative medicine.

### Exemplification of differentiation of stem cells to intestinal stem cells

Stem cells (e.g. iPS or HES cells) can be differentiated in standard culture to intestinal stem cells according to a protocol described by Loh *et al* (*supra*).

Supplying the ligands (including those that cannot be added to the medium like Notch ligands) to the cell culture in a controlled manner in accordance with an embodiment of the present invention to activate the respective signaling pathways that are assumed to become active during various differentiation stages (cf. Figure 1, pathway analysis of a GEO dataset, differentiation to hindgut; A. pathway activities measured per differentiation step; B. overview picture shows sequential differentiation steps; signaling pathway activities are indicated as log2 odds values per individual sample) improves the outcome of the differentiation experiment and will make such experiments more reproducible across labs and time and render the method in particular useful for standardization purposes of the experiment.

An exemplary experiment in accordance with the present invention is carried out as follows. The experiment is designed that the cells go through several differentiation steps, first to obtain anterior primitive streak cells, subsequently definitive endoderm, and then mid/hindgut cells phenotype.

Stem cells are cultured feeder-free at the required density in defined serum-free CDM2 basal medium on tissue culture plastic. Subsequently for the differentiation experiment they are passaged and seeded on the herein described surface material that is functionalized with a 50/50 mixture (10 microgram/ml) of Activin, an appropriate Wnt ligand (e.g. Wnt 3A), in the presence of an mTOR inhibitor in the CDM2 medium, e.g. LDN-193189/DM3189, (50 nM), for 24 hours to obtain anterior primitive streak cells. Subsequently, cells are detached from the surface and pathway analysis is performed on a cell sample for all mentioned signal transduction pathways, to ascertain that the cells reached the required differentiation step to a required quantitative extent. When this pathway analysis produces satisfactory results, this enables a positive decision to continue with the experiment. Cells are subsequently seeded onto a next substrate functionalized with Activin in the presence of the BMP-inhibitor LDN-193189/DM3189 (250 nM) in CDM2 for 48 hours to obtain definitive endoderm. Cells are detached again, differentiation status again measured in a quantitative manner using pathway analysis for all mentioned signal transduction pathways. If confirmative, cells are reseeded and cultured on a herein described surface material functionalized with a 15/15/35/35 mixture (10 microgram/ml) of BMP4, FGF2 and high Wnt ligand (Wnt 3A) and Notch ligand (e.g. Delta) for 4 days in CDM2 medium containing retinoic acid (2 µM, Sigma) and BMP-inhibitor DM3189 (250 nM), optionally with the differentiating agent methoxy substituted alkylglycerol-1-O-(2-methoxy) hexadecyl glycerol (MHG) added, for 4 days. Media is refreshed every 24 hours. At the end, cells are detached form the surface and pathway analysis can be performed once more to ascertain that the required final differentiation step has been obtained and to quantify the final result (e.g. active Wnt and Notch pathway).

### Exemplification of functionalizing a surface with a cell receptor ligand

An experiment designed as proof of principle for coupling a cell receptor ligand to a functionalized surface is described using TGFbeta as a representative example. The reason TGFbeta was chosen is that the effect of TGFbeta can be shown by adding TGFbeta to the medium as well, enabling performing a control experiment for the effect of TGFbeta on the used cultured cells. While the described method provides a large advantage over currently used culture methods when coating the functionalized material with a Hedgehog (HH) or Notch ligand, a control experiment with these ligands would not have been possible since these ligands preferably or only act when attached to a surface. However when coating of the functionalized surface with TGFbeta successfully leads to induction of TGFBeta pathway activity in attached cells, it can be safely concluded that coating with other protein ligands will be similarly effective. TGFb1 was commercially bought from STEMCELL TECHNOLOGIES INC. The TGFbeta coating experiment was performed as follows: WPMY1 fibroblast cells were seeded and cultured (DMEM medium with 10% FBS) on functionalized surface (SIL-IM-F 0.5%, silicone substrate containing linoleic acid as described herein and in international patent application PCT/EP2018/068318 referred to above), which was according to described protocol coated with TGFb1 by incubation with 10 ug TGFb1 (SIL-IM-F 0.5%-TGFb1), or as control non functionalized (SIL-IM-NF) surface, similarly incubated with TGFb1 (SIL-IM-NF 0.5%). In another control situation, WPMY1 cells were cultured on standard tissue culture plastic and 1 ng/ml TGFb1 was added to the medium. Cells were cultured for 48 hours, released from the surface and signaling pathway analysis was performed on RNA isolated from the cells as described herein and the patent application referred to above in the context of determining activity of signaling pathways. Cells attached and proliferated on all surfaces. A response to TGFBeta was measured as a higher TGFbeta pathway score in the standard culture (on culture plastic) compared to unstimulated WPMY1 cells (52% higher), serving as control for responsiveness to stimulation with TGFb1 in these cells (Table 1). TGFb1 coating of functionalized surface resulted in 103% increase in TGFbeta pathway activity; TGFb1 coating of non-functionalized surface resulted in only 67% increase in TGFbeta activity (cf. Table 1).

**Table 1: Comparison of activation of TGFbeta pathway by spiking TGFb1 or coating a non-functionalized and functionalized surface with TGFb1.**

| **TGFB response With vs without TGFb1** | % increase |
|---|---|
| control (medium spiked) | 52% |
| SIL-IM-NF (non-functionalized surface; coated) | 67% |
| SIL-IM-F 0.5% (functionalized; coated) | 103% |

This shows that the functionalized surface when coated with TGFb1 was more effective in stimulating the TGFbeta pathway in WPMY1 cells that are responsive to the TGFbeta ligand. In agreement with this, the cell morphology changed compared to cells on the non-TGFbl-coated surface (cf. Figure 2).

In an independent cell culture experiment the tissue culture-treated plastic surface, non-functionalized and functionalized surface were incubated with different concentrations of TGFb1, 0 ng/ml, 10 ng/ml and 1000 ng/ml (Figure 3, indicated as respectively NO, 10 ng and 1 microgram; on the y-axis is the TGFBeta pathway activity score), and TGFbeta pathway activity was analyzed. TGFbeta pathway activity increased with the concentration of TGFb1 with which the functionalized surface had been incubated to couple the TGFb1 to the COOH groups on the functionalized surface. The increase was comparable to the effect seen when cells were treated with 1 ng/ml TGFb1 in the culture medium, when cultured on tissue culture-treated plastic. In contrast to the non-functionalized surface a similar incubation with TGFb1 did not increase TGFbeta pathway activity (Figure 3).

## Claims

1. *In vitro* method of producing a cell culture, tissue or organ, the method comprising:
(a) determining first culture conditions that control activity of at least one first signaling pathway of cells to be cultivated, the activity of the at least one first signaling pathway being selected so to control cell behavior in a first predetermined manner;
(b) cultivating cells in a culture unit under the first culture conditions to obtain a cell culture;
(c) inferring activity of the at least one first signaling pathway in a cell sample obtained from the cell culture; and
(d) comparing the inferred activity with the selected activity.

2. The method according to claim 1, the method further comprising:
(e) determining second culture conditions based on the comparison of the inferred activity with the selected activity.

3. The method according to claim 2,
wherein the second culture conditions are determined so as to (i) reduce deviation of the inferred activity from the selected activity, or (ii) control activity of at least a second signaling pathway, the activity of the at least one second signaling pathway being selected so to control cell behavior in a second predetermined manner.

4. The method according to claim 2 or 3, further comprising:
(f) cultivating the cells in the culture unit under the second culture conditions;
(g) repeating steps (c) and (d) as defined in claim 1; and optionally
(h) determining third culture conditions based on the comparison of the activity inferred in step (g) with the selected activity.

5. The method according to any one of the preceding claims,
wherein the cell behavior is selected from the group consisting of differentiation, maintenance of pluripotency and cellular function, such as cell division, migration and metabolism, chemotaxis, adhesion, apoptosis, generating oxidative stress, DNA repair, production of secretory proteins, production of extracellular matrix proteins etc.

6. The method according to any one of the preceding claims,
wherein the first and/or second culture conditions are defined by controlled exposure of the cells to one or more stimuli selected from the group consisting of cell receptor ligands, extracellular matrix molecules, artificial receptor agonists or antagonists, and specific antigens for T or B cell receptors, the stimuli controlling activity of the at least one first and/or second signaling pathway and optionally being coupled to a surface of the culture unit.

7. The method according to claim 6,
wherein the surface comprises or consists of a polymer-based material, preferably based on polydimethylsiloxane (PDMS) or rubber or silicone, and wherein the one or more stimuli are coupled to the surface.

8. The method according to claim 7,
wherein the one or more stimuli are coupled to the surface via carboxyl groups.

9. The method according to any one of the preceding claims,
wherein the cells are mouse stem cells, human stem cells, pluripotent stem cells, mesenchymal stem cells, embryonic stem cells, adult stem cells, tumor cells, cell lines.

10. The method according to any one of the preceding claims,
wherein the culture unit is a microfluidic device,
wherein the microfluidic device preferably comprises a polymer-based material as defined in claim 7 or claim 8 stacked between a top cover plate and a bottom cover plate and
wherein the membrane is optionally adapted to adhere to the top cover plate and/or the bottom cover plate.

11. The method according to any one of the preceding claims,
wherein the at least one signaling pathway is selected from the group of nuclear receptors, like ER, AR, progesterone receptor; growth factor pathways, like PI3K-FOXO, JAK-STAT3, MAPK-AP1 pathways; immune pathways like STAT1/2 type I interferon (STAT1/2-1), STAT1/2 type II interferon (STAT1/2-2) pathways; developmental pathways, like Hedgehog, Notch, TGFbeta, and Wnt signaling pathways; and inflammatory pathway NFkB.

12. The method according to any one of the preceding claims,
wherein the activity of the at least one first and/or second signaling pathway in the cell sample is inferable by a method comprising:
receiving expression levels of three or more target genes of the at least one signaling pathway in at least one cell contained in the cell sample,
determining an activity level of a transcription factor (TF) element in the at least one cell, the TF element controlling transcription of the three or more target genes, the determining being based on evaluating a calibrated mathematical pathway model relating expression levels of the three or more target genes to the activity level of the at least one signaling pathway, and
inferring the activity of the at least one signaling pathway in the at least one cell based on the determined activity level of the TF element,
wherein the calibrated mathematical pathway model is preferably a centroid, linear or Bayesian network model, based on conditional probabilities.

13. A non-transitory storage medium storing instruction that are executable by a digital processing device to perform the method of any of claims 1 to 12.

14. A computer program comprising program code means for causing a digital processing device to perform the method of any of claims 1 to 12, when the computer program is run on a digital processing device.

15. A system for performing the method of any of claims 1 to 11, the system comprising
(a) a culture unit,
(b) a sensor unit for inferring an activity of at least one first signaling pathway;
(c) a controller that is configured to compare the inferred activity with a predetermined activity of the at least one first signaling pathway, the predetermined activity being selected so to control cell behavior in a first predetermined manner and optionally
(d) a non-transitory storage medium according to claim 13 or a computer program according to claim 14.

16. The system of claim 15,
wherein the controller is further configured to determine culture conditions based on the comparison of the inferred activity with the predetermined activity, the culture conditions being optionally determined so as to (ii) reduce deviation of the inferred activity from the predetermined activity or (ii) control activity of at least one second signaling pathway, the activity of the at least one second signaling pathway.
